# EUROPÄISCHE PATENTSCHRIFT

(11) **EP 0 863 886 B1**
(45) Veröffentlichungstag und Bekanntmachung des Hinweises auf die Patenterteilung: **09.01.2002**
(21) Anmeldenummer: 96934558.6
(22) Anmeldetag: 07.10.1996
(51) Int. Cl.: C07D 285/08, C07D 417/04, A01N 43/836, C07C 69/734, C07D 307/12, C07D 309/12

(54) **FLUORMETHOXYACRYLSÄUREDERIVATE UND IHRE VERWENDUNG ALS SCHÄDLINGSBEKÄMPFUNGSMITTEL**
FLUOROMETHOXYACRYLIC ACID DERIVATIVES AND THEIR USE AS PEST CONTROL AGENTS
DERIVES D'ACIDE FLUOROMETHOXYACRYLIQUE ET LEUR UTILISATION EN TANT QUE PESTICIDES

(30) Priorität: 18.10.1995 DE 19538790; 25.03.1996 DE 19611653
(43) Veröffentlichungstag der Anmeldung: 16.09.1998
(60) Teilanmeldung: 00118137.9
(73) Patentinhaber: BAYER AG, 51368 Leverkusen (DE)
(72) Erfinder: HEINEMANN, Ulrich, D-42799 Leichlingen (DE); GAYER, Herbert, D-40789 Monheim (DE); GERDES, Peter, D-52080 Aachen (DE); MARHOLD, Albrecht, D-51373 Leverkusen (DE); STELZER, Uwe, D-51399 Burscheid (DE); TIEMANN, Ralf, D-51375 Leverkusen (DE); STENZEL, Klaus, D-40595 Düsseldorf (DE); DUTZMANN, Stefan, D-40721 Hilden (DE)
(86) Internationale Anmeldenummer: EP9604344
(87) Internationale Veröffentlichungsnummer: WO9714687

(56) Entgegenhaltungen:
- WO-A-94/10159
- WO-A-95/17376

## Beschreibung

Die Erfindung betrifft neue Fluormethoxyacrylsäurederivate, ein Verfahren zu ihrer Herstellung und ihre Verwendung als Schädlingsbekämpfungsmittel, sowie neue Zwischenprodukte und mehrere Verfahren zu deren Herstellung.

Es ist bereits bekannt geworden, dass bestimmte Fluormethoxyacrylsäurederivate die den unten beschriebenen Verbindundungen konstitutionell ähnlich sind, füngizide Eigenschaften besitzen (vergleiche z. B. WO 9517376). Die füngizide Wirkung dieser Verbindungen ist jedoch in vielen Fällen unbefriedigend.

Es wurden nun die neuen Fluormethoxyacrylsäurederivate der allgemeinen Formel (Ia) gefunden, 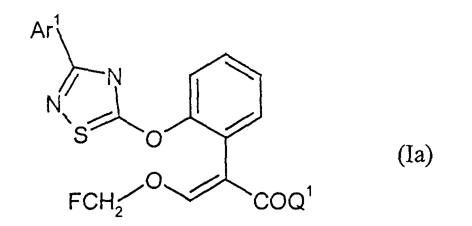 in welcher

| | | | |
|---|---|---|---|
| Ar¹ | für 4-Bromphenyl | und Q¹ | für Methoxy, oder |
| Ar¹ | für 4-Methylphenyl | und Q¹ | für Methoxy, oder |
| Ar¹ | für 4-Methoxyphenyl | und Q¹ | für Methoxy, oder |
| Ar¹ | für 4-Chlorphenyl | und Q¹ | für Methoxy, oder |
| Ar¹ | für 4-Fluorphenyl | und Q¹ | für Methoxy, oder |
| Ar¹ | für 3-Methylphenyl | und Q¹ | für Methoxy, oder |
| Ar¹ | für 2-Methylphenyl | und Q¹ | für Methoxy, oder |
| Ar¹ | für 3-Fluorphenyl | und Q¹ | für Methoxy, oder |
| Ar¹ | für 2-Fluorphenyl | und Q¹ | für Methoxy, oder |
| Ar¹ | für 4-Bromphenyl | und Q¹ | für Ethoxy, oder |
| Ar¹ | für 2,4-Dimethylphenyl | und Q¹ | für Methoxy, oder |
| Ar¹ | für 3,4-Dichlorphenyl | und Q¹ | für Methoxy, oder |
| Ar¹ | für 4-Trifluormethylphenyl | und Q¹ | für Methoxy, oder |
| Ar¹ | für 3-Chlorphenyl | und Q¹ | für Ethoxy, oder |
| Ar¹ | für 2-Chlorphenyl | und Q¹ | für Methoxy, oder |
| Ar¹ | für 2-Bromphenyl | und Q¹ | für Methoxy, oder |
| Ar¹ | für 2-Methoxyphenyl | und Q¹ | für Methoxy, oder |
| Ar¹ | für 3-Chlorphenyl | und Q¹ | für Methoxy, |

steht.

Die erfindungsgemäßen Verbindungen können gegebenenfalls als Mischungen verschiedener möglicher isomerer Formen, insbesondere von Stereoisomeren, wie z. B. E- und Z-, vorliegen. Es werden sowohl die E- als auch die Z-Isomeren, wie auch beliebige Mischungen dieser Isomeren, beansprucht.

In den Definitionen sind die gesättigten oder ungesättigten Kohlenwasserstoffketten, wie Alkyl jeweils geradkettig oder verzweigt.

Die oben aufgeführten Restedefinitionen gelten sowohl für die Endprodukte der Formel (I) als auch entsprechend für die jeweils zur Herstellung benötigten Ausgangsstoffe bzw. Zwischenprodukte.

Weiterhin wurde gefunden, dass die Fluormethoxyacrylsäurederivate der Formel (I) sehr gute mikrobizide Eigenschaften besitzen und zum Schutz von Pflanzen gegen Schadorganismen eingesetzt werden können.

Überraschenderweise zeigen die erfindungsgemäßen Stoffe eine bessere Wirkung als konstitutionell ähnliche, vorbekannte Wirkstoffe gleicher Wirkungsrichtung.

Schließlich wurde gefunden, dass man die neuen Fluormethoxyacrylsäurederivate der allgemeinen Formel (Ia), 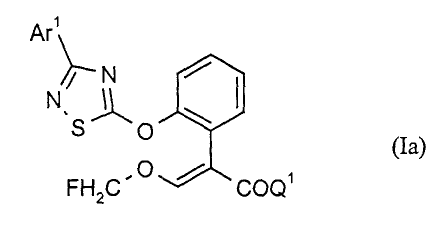 in welcher
- Ar¹ und Q¹: die oben angegebenen Bedeutungen haben
erhält, wenn man (Verfahren a) Hydroxyarylverbindungen der allgemeinen Formel (II), 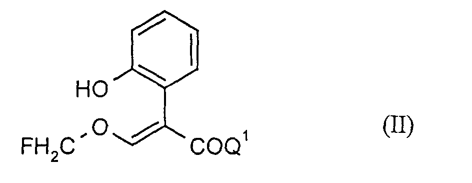 in welcher
- Q¹: die oben angegebenen Bedeutungen hat,
mit einemThiadiazolderivat der allgemeinen Formel (III), 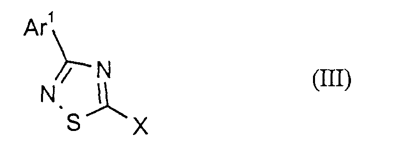 in welcher
- Ar¹: die oben angegebene Bedeutung hat und
- X: für Fluor, Chlor, Methylsulfonyl, Benzylsulfonyl oder Tolylsulfonyl steht,
gegebenenfalls in Gegenwart eines Säureakzeptors und gegebenenfalls in Gegenwart eines Verdünnungsmittels umsetzt.

Dabei ist als besonders überraschend anzusehen, dass das erfindungsgemäße Verfahren mit erheblich höheren Ausbeuten, als das nach dem Stand der Technik bekannte Verfahren verläuft.

Die zur Durchführung des erfindungsgemäßen Verfahrens a) als Ausgangsstoffe benötigten Hydroxyarylverbindungen sind durch die Formel (II) allgemein definiert. In dieser Formel (II) hat Q¹ vorzugsweise bzw. insbesondere diejenige Bedeutung, die bereits im Zusammenhang mit der Beschreibung der erfindungsgemäßen Verbindungen der Formel (Ia) als bevorzugt bzw. als insbesondere bevorzugt für Q¹ angegeben wurde.

Die Hydroxyarylverbindungen der Formel (II) sind noch nicht bekannt; sie sind als neue Stoffe Gegenstand der vorliegenden Anmeldung.

Die Hydroxyarylverbindungen der Formel (II) werden erhalten (Verfahren b), wenn man Acetale der Formel (IV), 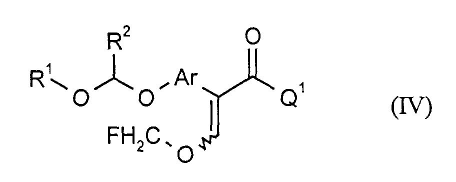 in welcher
- R¹: für Alkyl steht,
- R²: für Wasserstoff oder Alkyl steht oder
- R¹ und R²: gemeinsam mit den Atomen an die sie gebunden sind, für einen fünf- oder sechsgliedrigen, heterocyclischen Ring stehen
- Q¹: die oben angegebene Bedeutung hat,
gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; eines Ketons, wie Aceton, Butanon, Methyl-isobutylketon oder Cyclohexanon; eines Nitrils, wie Acetonitril, Propionitril, n- oder i-Butyronitril oder Benzonitril; eines Esters wie Essigsäuremethylester oder Essigsäureethylester; eines Sulfoxides, wie Dimethylsulfoxid; eines Sulfons, wie Sulfolan; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemischen mit Wasser oder reinem Wasser und gegebenenfalls in Gegenwart einer Säure, vorzugsweise einer anorganischen oder organischen Protonen- oder Lewissäure, wie beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Zinkchlorid, Eisen-III-chlorid, Antimonpentachlorid, oder auch einer polymeren Säure wie beispielsweise einem sauren Ionenaustauscher, einer sauren Tonerde oder saurem Kieselgel, bei Temperaturen von -20°C bis 120°C, vorzugsweise bei Temperaturen von -10°C bis 80°C, hydrolysiert.

Die zur Durchführung des erfindungsgemäßen Verfahrens b) als Ausgangsstoffe benötigten Acetale sind durch die Formel (IV) allgemein definiert. In dieser Formel (IV) hat Q¹ vorzugsweise bzw. insbesondere diejenige Bedeutung, die bereits im Zusammenhang mit der Beschreibung der erfindungsgemäßen Verbindungen der Formel (Ia) als bevorzugt bzw. als insbesondere bevorzugt für Q¹ angegeben wurde. R¹ steht für Alkyl, vorzugsweise für Methyl oder Ethyl, R² steht für Wasserstoff oder Alkyl, vorzugsweise für Methyl oder Ethyl, oder R¹ und R² stehen gemeinsam mit den Atomen an die sie gebunden sind, für einen fünf- oder sechsgliedrigen, heterocyclischen Ring, vorzugsweise für Tetrahydrofuryl oder Tetrahydropyryl.

Die Acetale der Formel (IV) sind noch nicht bekannt; sie sind als neue Stoffe Gegenstand der vorliegenden Anmeldung.

Die Acetale der Formel (IV) werden erhalten, wenn man (Verfahren c) Arylessigsäurederivate der Formel (V), 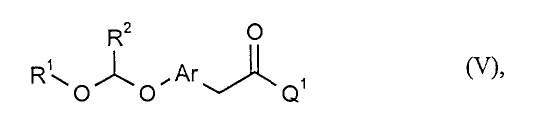 in welcher
R¹, R² und Q¹ die oben angegebenen Bedeutungen haben,
zunächst mit einem Ameisensäurederivat, wie beispielsweise Ameisensäuremethylester, Kohlenmonoxid, eines Dialkylformamidacetals oder eines Bis-dialkylaminoalkoxymethans gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; eines Ketons, wie Aceton, Butanon, Methyl-isobutylketon oder Cyclohexanon; eines Nitrils, wie Acetonitril, Propionitril, n- oder i-Butyronitril oder Benzonitril; eines Esters wie Essigsäuremethylester oder Essigsäureethylester; eines Amids, wie N,N-Dimethylformamid, N,N-Dimethylacetamid, N-Methylformanilid, N-Methylpyrrolidon oder Hexamethylphosphorsäuretriamid; eines Sulfoxides, wie Dimethylsulfoxid; eines Sulfons, wie Sulfolan; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether oder Diethylenglykolmonoethylether, und gegebenenfalls in Gegenwart eines basischen Katalysators, vorzugsweise eines Erdalkalimetall- oder Alkalimetallhydrides, -hydroxides, -amides, -alkoholates, -acetates, -carbonates oder -hydrogencarbonates, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiären Amines, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), bei Temperaturen von -20°C bis 120°C, vorzugsweise bei Temperaturen von -10°C bis 80°C, umsetzt, und die so erhaltenen Enole der Formel (VI), 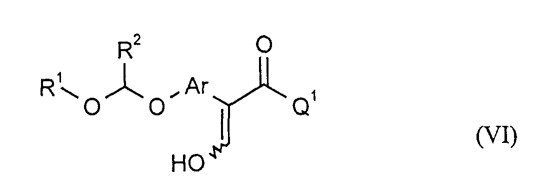 in welcher
R¹, R² und Q¹ die oben angegebenen Bedeutungen haben,
vorzugsweise ohne weitere Aufarbeitung mit Fluorbrommethan oder Fluorchlormethan, gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofüran, 1,2-Dimethoxyethan, 1,2-Diethoxyethan oder Anisol; eines Ketons, wie Aceton, Butanon, Methyl-isobutylketon oder Cyclohexanon; eines Nitrils, wie Acetonitril, Propionitril, n- oder i-Butyronitril oder Benzonitril; eines Esters wie Essigsäuremethylester oder Essigsäureethylester; eines Amids, wie N,N-Dimethylformamid, N,N-Dimethylacetamid, N-Methylformanilid, N-Methylpyrrolidon oder Hexamethylphosphorsäuretriamid; eines Sulfoxides, wie Dimethylsulfoxid; eines Sulfons, wie Sulfolan; eines Alkohols, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemischen mit Wasser oder reinem Wasser, und gegebenenfalls in Gegenwart einer Base, vorzugsweise eines Erdalkalimetall- oder Alkalimetallhydrides, -hydroxides, -amides, -alkoholates, -acetates, -carbonates oder -hydrogencarbonates, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, oder eines tertiären Amines, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), bei Temperaturen von -20°C bis 120°C, vorzugsweise bei Temperaturen von -10°C bis 80°C, umsetzt.

Die zur Durchführung der erfindungsgemäßen Verfahren c) zur Herstellung der Acetale der Formel (IV) als Ausgangsstoffe benötigten Arylessigsäurederivate sind durch die Formel (V) allgemein definiert. In dieser Formel (V) hat Q¹ vorzugsweise bzw. insbesondere diejenige Bedeutung, die bereits im Zusammenhang mit der Beschreibung der erfindungsgemäßen Verbindungen der Formel (Ia) als bevorzugt bzw. als insbesondere bevorzugt für Q¹ angegeben wurde. R¹ und R² haben vorzugsweise diejenigen Bedeutungen, die bereits im Zusammenhang mit der Beschreibung der Verbindungen der Formel (IV) als bevorzugt angegeben wurden.

Die Arylessigsäurederivate der Formel (V) sind teilweise bekannt und/oder können nach bekannten Verfahren hergestellt werden (vergleiche z. B. J. Org. Chem. 1994, 203-13).

Neu und auch Gegenstand der vorliegenden Anmeldung sind die Arylessigsäurederivate der Formel (Va), 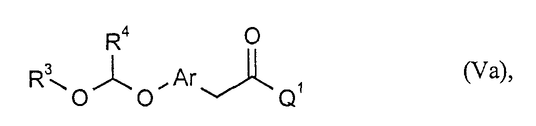 in welcher
- R³ und R⁴: gleich oder verschieden sind und unabhängig voneinander für Alkyl stehen und
- Q¹: die oben angegebene Bedeutung hat, und
- Ar: für o-Phenylen steht.

Die Arylessigsäurederivate der Formel (Va) werden erhalten (Verfahren d), wenn man Hydroxyverbindungen der allgemeinen Formel (VII), 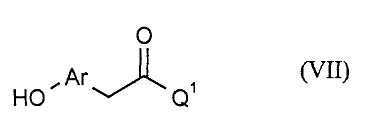 in welcher
- Q¹: die oben angegebene Bedeutung hat,
mit Vinylethern der allgemeinen Formel (VIII) 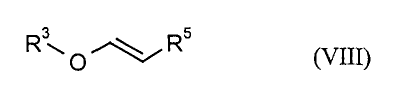 in welcher
- R³: die oben angegebene Bedeutung hat und
- R⁵: für Wasserstoff oder Alkyl steht,
gegebenenfalls in Gegenwart eines Verdünnungsmittels, vorzugsweise eines aliphatischen, alicyclischen oder aromatischen Kohlenwasserstoffes, wie beispielsweise Petrolether, Hexan, Heptan, Cyclohexan, Methylcyclohexan, Benzol, Toluol, Xylol oder Decalin; eines halogenierten Kohlenwasserstoffes, wie beispielsweise Chlorbenzol, Dichlorbenzol, Dichlormethan, Chloroform, Tetrachlormethan, Dichlorethan oder Trichlorethan; eines Ethers, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol oder eines Nitrils, wie Acetonitril, Propionitril, n- oder i-Butyronitril oder Benzonitril, und gegebenenfalls in Gegenwart einer Säure durchgeführt, vorzugsweise einer anorganischen oder organischen Protonen- oder Lewissäure, oder auch einer polymeren Säure, wie beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Zinkchlorid, Eisen-III-chlorid, Antimonpentachlorid, sauren Ionenaustauschern, sauren Tonerden und saurem Kieselgel, umsetzt.

Die neuen Arylessigsäurederivate sind durch die Formel (Va) allgemein definiert. In dieser Formel (Va) hat Q¹ vorzugsweise bzw. insbesondere diejenige Bedeutung, die bereits im Zusammenhang mit der Beschreibung der erfindungsgemäßen Verbindungen der Formel (Ia) als bevorzugt bzw. als insbesondere bevorzugt für Q¹ angegeben wurde. R³ und R⁴ sind gleich oder verschieden und stehen unabhängig voneinander für Alkyl, vorzugsweise für Methyl oder Ethyl.

Die zur Durchführung des erfindungsgemäßen Verfahrens d) zur Herstellung der erfindungsgemäßen Verbindungen der Formel (Va) benötigten Hydroxyverbindungen sind durch die Formel (VII) allgemein definiert. In dieser Formel (VII) hat Q¹ vorzugsweise bzw. insbesondere diejenige Bedeutung, die bereits im Zusammenhang mit der Beschreibung der erfindungsgemäßen Verbindungen der Formel (Ia) als bevorzugt bzw. als insbesondere bevorzugt für Q¹ angegeben wurde.

Die Hydroxyverbindungen der Formel (VII) sind bekannte Synthesechemikalien.

Die zur Durchführung des erfindungsgemäßen Verfahrens d) zur Herstellung der erfindungsgemäßen Verbindungen der Formel (Va) weiterhin benötigten Vinylether sind durch die Formel (VIII) allgemein definiert. In dieser Formel (VII) hat R³ vorzugsweise diejenige Bedeutung, die bereits im Zusammenhang mit der Beschreibung der erfindungsgemäßen Verbindungen der Formel (Va) als bevorzugt bzw. als insbesondere bevorzugt für R³ angegeben wurde. R⁵ steht für Wasserstoff oder Alkyl, vorzugsweise für Wasserstoff oder Methyl.

Die Vinylether der Formel (VIII) sind bekannte Synthesechemikalien.

Die zur Durchführung des erfindungsgemäßen Verfahrens a) weiterhin als Ausgangsstoffe benötigten Thiadiazolderivate sind durch die Formel (III) allgemein definiert. In dieser Formel (III) hat Ar¹ die oben angegebenen Bedeutungen. X steht für Fluor oder Chlor, Methylsulfonyl, Benzylsulfonyl oder Tolylsulfonyl.

Die Thiadiazolderivate der Formel (III) sind bekannte Synthesechemikalien und/oder können nach bekannten Verfahren hergestellt werden (vergleiche z. B. J. Heterocyclic Chem. 30, 357 (1993)).

Als Verdünnungsmittel zur Durchführung der erfindungsgemäßen Verfahren a) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren a) wird gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens a) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens a) zur Herstellung der Verbindungen der Formel (Ia) setzt man pro Mol der Hydroxyarylverbindungen der allgemeinen Formel (II) im allgemeinen 0,2 bis 5 Mol, vorzugsweise 0,5 bis 2 Mol an Thiadiazolderivat der allgemeinen Formel (III) ein.

Das erfindungsgemäße Verfahren a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach bekannten Verfahren (vgl. auch die Herstellungsbeispiele).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei werden die erfindungsgemäßen Wirkstoffe mit besonders gutem Erfolg zur Bekämpfung von Getreidekrankheiten, wie beispielsweise gegen Erysiphe- oder Leptosphaeria-Arten, oder von Krankheiten im Wein-, Obst- und Gemüseanbau, wie beispielsweise gegen Plasmopara-, Uncinula-, Sphaerotheca- oder Venturia-Arten, oder auch von Reiskrankheiten, wie beispielsweise Pyricularia-Arten, eingesetzt. Außerdem zeigen die erfindungsgemäßen Wirkstoffe eine besonders starke und breite in vitro Wirkung.

Die Wirkstoffe werden in Abhängigkeit von ihren jeweiligen physikalischen und/oder chemischen Eigenschaften in die üblichen Formulierungen übergeführt, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

In vielen Fällen werden dabei synergistische Wirkungen beobachtet.

Besonders günstige Mischpartner sind z.B. die folgenden Verbindungen:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazole-5-carboxanilid; 2,6-Dichloro-N-(4-trifluoromethylbenzyl)benzamid; (E)-2-Methoxyimino-N-methyl-2-(2-phenoxyphenyl)-acetamid; 8-Hydroxychinolinsulfat; Methyl-(E)-2- {2-[6-(2-cyanophenoxy)-pyrimidin-4-yloxy]-phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung, Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol, Validamycin A, Vinclozolin,
Zineb, Ziram

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chloretoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron ,Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Pirimiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe werden als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verschäumen, Bestreichen usw.. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die erfindungsgemäßen Wirkstoffe werden als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verschäumen, Bestreichen usw.. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Beispiele

### Beispiel 1

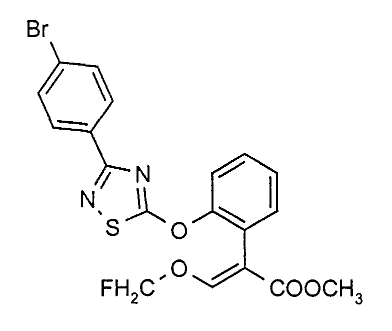

### Verfahren a)

Eine Lösung von 3,2 g (0,01 Mol) 3-Phenyl-5-toluolsulfonyl-1,2,4-thiadiazol und 1,1 g 2-(2-Hydroxyphenyl)-3-fluormethoxy-acrylsäuremethylester in 30 ml trockenem Dimethylformamid wird bei 0°C mit 0,3 g (0,01 Mol) 80%igem Natriumhydrid in Mineralöl versetzt. Die Mischung wird 3 Tage ohne Kühlung gerührt und anschließend eingeengt. Der Rückstand wird mit Essigsäureethylester aufgenommen, mit Wasser gewaschen, die organische Phase über Natriumsulfat getrocknet und wiederum eingeengt. Das Rohprodukt wird mit Hexan/Aceton (9:1) an Kieselgel chromatografiert. Man erhält 2,8 g (60 % der Theorie) 2-{2-[3-(4-Bromphenyl)-1,2,4-thiadiazol-5-yloxy]-phenyl}-3-fluormethoxy-acrylsäuremethylester als gelbes, hochviskoses Öl.

¹H-NMR (CDCl₃, TMS): δ = 5,4 (d, 2H) ppm.

### Herstellung des Ausgangsstoffes

### Beispiel II-1

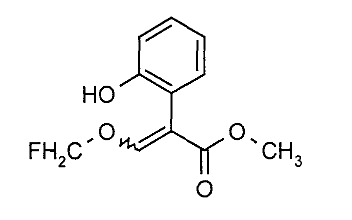

### Verfahren b)

6,2 g (0,02 Mol) 2-[2-(Tetrahydropyran-2-yloxy)-phenyl]-3-fluormethoxy-acrylsäuremethylester werden in 30 ml Methanol mit 0,5 g saurem Ionenaustauscher bei 20°C 18 Stunden gerührt. Der Ionenaustauscher wird abfiltriert und das Filtrat eingeengt. Der Rückstand wird mit Hexan/Aceton (7:3) an Kieselgel chromatografiert. Man erhält 3,3 g (73 % der Theorie) 2-(2-Hydroxyphenyl)-3-fluormethoxy-acrylsäuremethylester als hellgelbes, viskoses Öl.

¹H-NMR (CDCl₃, TMS): δ = 5,4 (d, 2H) ppm.

Anstelle von 2-[2-(Tetrahydropyran-2-yloxy)-phenyl]-3-fluormethoxy-acrylsäuremethylester kann auch 2-[2-(1-Ethoxy-ethoxy)-phenyl-3-fluormethoxy-acrylsäuremethylester verwendet werden.

Analog Beispiel II-1 sowie entsprechend der allgemeinen Beschreibung des erfindungsgemäßen Herstellungsverfahrens b), erhält man auch ) 2-(2-Hydroxyphenyl)-3-fluormethoxy-acrylsäureethylester.

¹H-NMR (CDCl₃, TMS): δ = 1,30 (t, 3H); 4,32 (q, 2H) ppm.

### Herstellung des Vorproduktes

### Beispiel IV-1

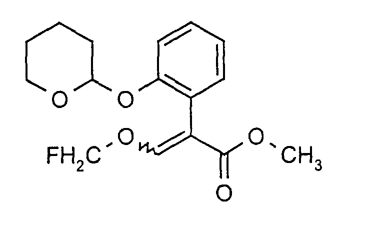

### Verfahren c)

In einem Dreihalskolben mit aufgesetztem Kühler werden einer Lösung von 12,5 g (60 mmol) 2-[2-(Tetrahydropyran-2-yloxy)-phenyl]-acrylsäuremethylester und 30 g Ameisensäuremethylester in 150 ml trockenem Dimethylformamid bei 20°C unter Rühren portionsweise 1,5 g 80%iges Natriumhydrid in Mineralöl gegeben. Nach etwa einer Stunde setzt die exotherme Reaktion unter Aufschäumen ein. Die Temperatur wird 3 Stunden lang bei 40°C gehalten. Die Mischung wird auf 0 - 5°C abgekühlt und nacheinander mit 4,8 g (0,05 Mol) Methansulfonsäure und portionsweise mit 13,8 g (0,1 Mol Kaliumcarbonat versetzt. Nun wird die Temperatur der Kühlflüssigkeit im Kühler, ebenso wie die Temperatur der Reaktionsmischung, auf 0°C eingestellt. Mit einer Spritze werden 6,8 g (0,06 mol) Bromfluormethan zugegeben und über Nacht bei 0-5°C gerührt. Die Reaktionsmischung wird weitere 24 Stunden ohne Kühlung weitergerührt. Die Temperatur der Kühlflüssigkeit wird solange bei etwa 0°C gehalten. Die Mischung wird eingeengt, der Rückstand wird mit Essigsäureethylester aufgenommen, mit Wasser gewaschen, die organische Phase über Natriumsulfat getrocknet und wiederum eingeengt. Der Rückstand wird mit Hexan/Aceton (7:3) an Kieselgel chromatografiert. Man erhält 11,6 g (75% der Theorie) 2-[2-(Tetrahydropyran-2-yloxy)-phenyl]-3-fluormethoxy-acrylsäuremethylester.

¹H-NMR (CDCl₃, TMS): δ = 5,5 (d, 2H) ppm.

Analog den Beispielen (1-2), sowie entsprechend der allgemeinen Beschreibung des erfindungsgemäßen Herstellungsverfahrens, erhält man auch die in der nachstehenden Tabelle 1 aufgeführten erfindungsgemäßen Verbindungen der Formel (Ia):

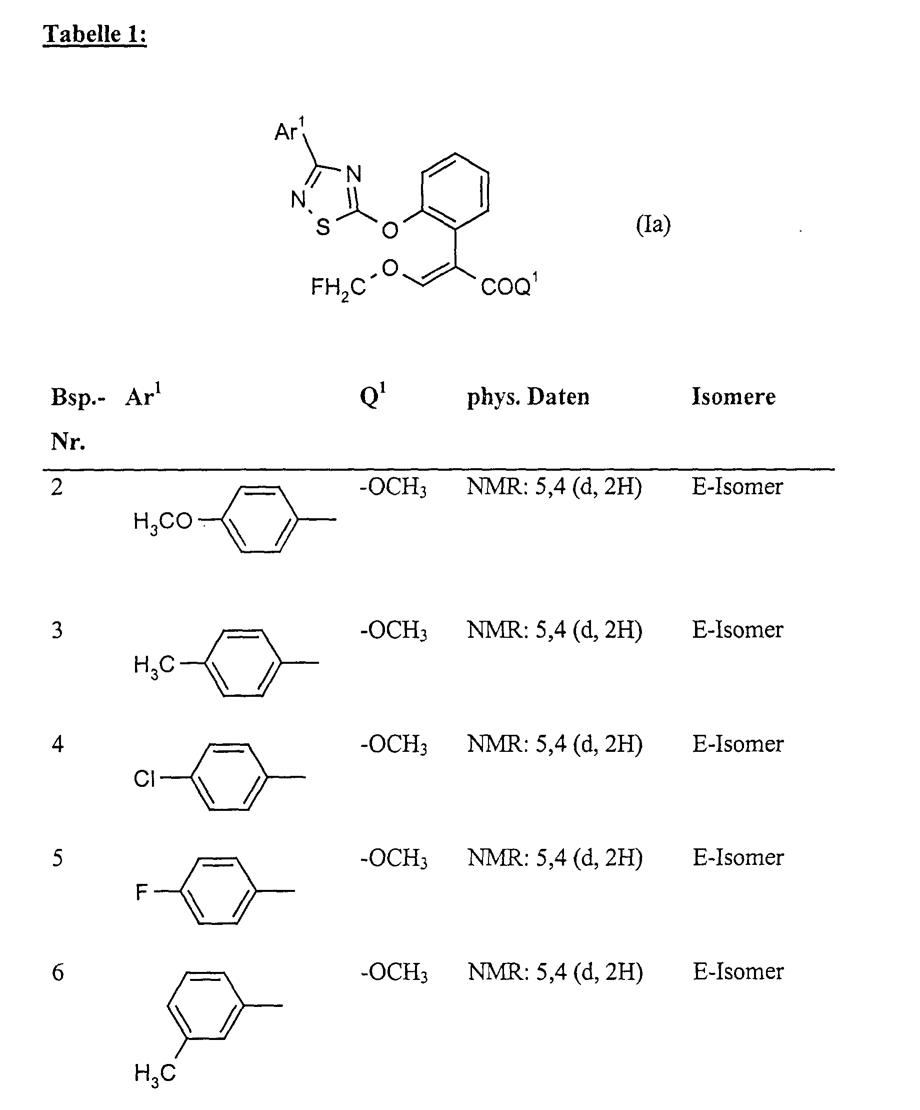

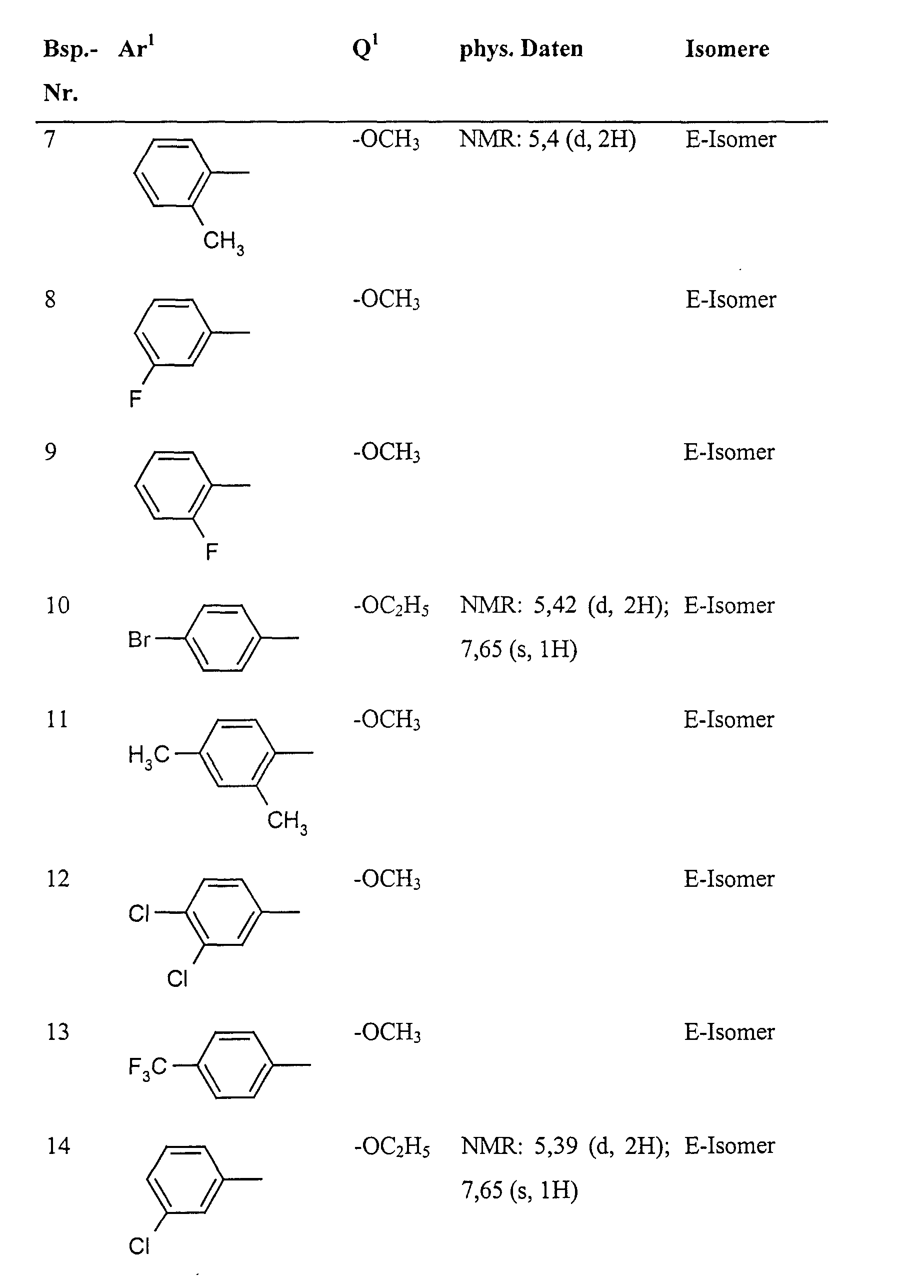

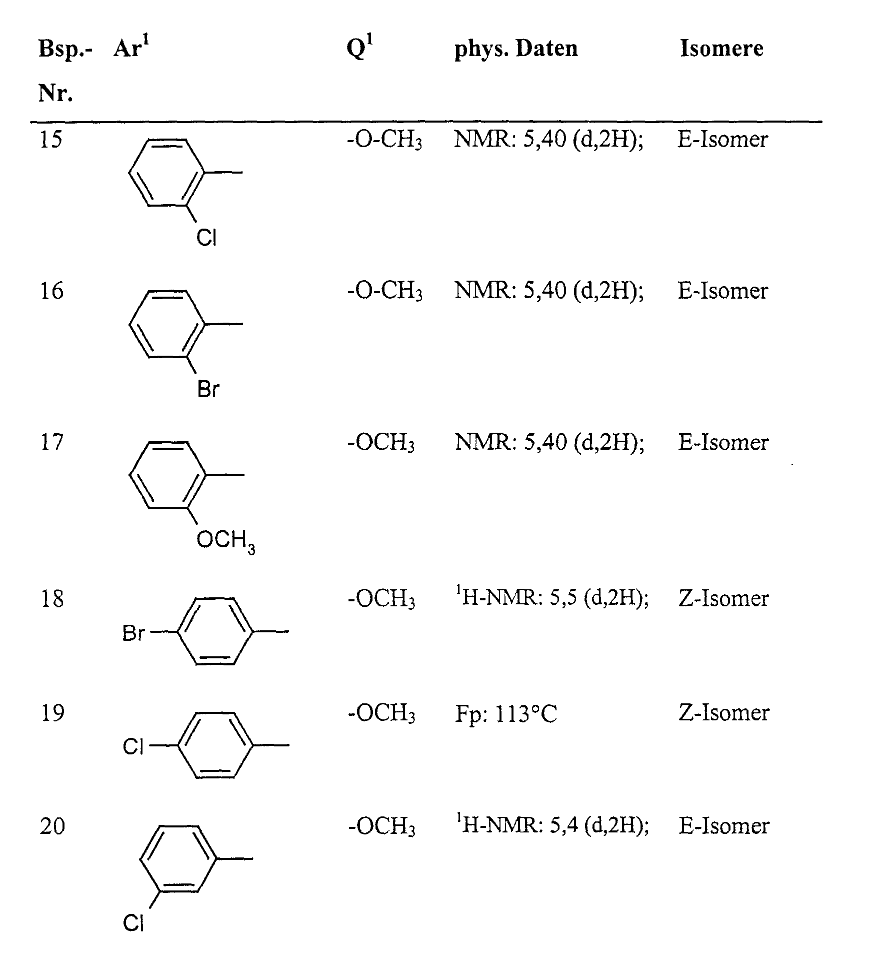

### Herstellung weiterer Vorprodukte

### Beispiel Va-1

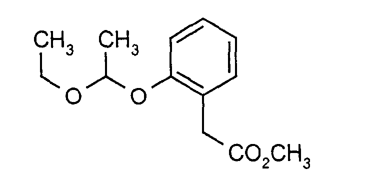

16,6 g (0,1 mol) 2-Hydroxyphenylessigsäuremethylester werden mit 10 g (0,13 mol) Ethylvinylether und 0,15 g p-Toluolsulfonsäure in 100 ml Methyl-tert.-butylether bei Raumtemperatur 5 Stunden gerührt. Die Lösung wird mit gesättigter Natriumhydrogencarbonat-Lösung gewaschen, über Natriumsulfat getrocknet und bei vermindertem Druck einggengt.

Man erhält 24,2 g (Gehalt: 94,5 % nach GC) [2-(1-Ethoxy-ethoxy)-phenyl]-essigsäuremethylester.

¹H-NMR (CDCl₃, TMS): δ = 1,19 (t, 3H); 1,48 (d, 3H); 3,68 (s, 3H).

### Beispiel IV-2

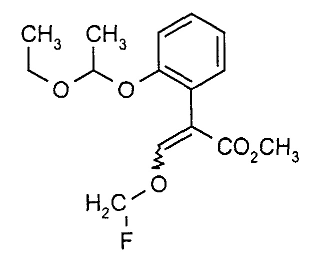

15,1 g (0,06 mol) [2-(1-Ethoxy-ethoxy)-phenyl]-essigsäure-methylester werden in 120 ml Dimethylformamid vorgelegt und mit 36 g (0,6 mol) Methylformiat bei Raumtemperatur versetzt. Bei 0°C werden 6,5 g Natriummethylat zugegeben und 3 Stunden bei dieser Temperatur gerührt. Dann erfolgt bei 0°C die Zugabe von 5,8 g (0,06 mol) Methansulfonsäure, 16,6 g (0,12 mol) Kaliumcarbonat und 7,4 g (0,065 ml) Bromfluor-methan. Man rührt das Reaktionsgemisch weitere 16 Stunden ohne Kühlung und gießt die Mischung auf 240 ml Wasser und 200 ml Toluol. Man gibt noch 12 g Natriumhydrogencarbonat zu und trennt die organische Phase ab. Diese Lösung wird über Natriumsulfat getrocknet und bei vermindertem Druck eingeengt.

Man erhält 18,1 g (Gehalt: 92,1 % nach GC bzw. HPLC) / E : Z-Anteil = 77 : 16) 2-[2-(Tetrahydropyran-2-yloxy)-phenyl]-3-fluormethoxy-acrylsäuremethylester.

¹H-NMR (CDCl₃) 100 \f "Symbol" \s 12d = 1,17 (t, 3H); 1,43 (d, 3H); 3,72 (s, 3H); 5,34-5,38 (m, 1H); 5,46 (d, 2H).
* ) Das ¹H-NMR-Spektrum wurde in Deuterochloroform (CDCl₃) oder Hexa-deuterodimethylsulfoxid (DMSO-d₆) mit Tetramethylsilan (TMS) als innerem Standard aufgenommen. Angegeben ist die chemische Verschiebung als d-Wert in ppm.

### Anwendungsbeispiele:

### Beispiel: A

### Plasmopara-Test (Reben) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wässrigen Sporensuspension von Plasmopara viticola inokuliert und verbleiben dann 1 Tag in einer Feuchtkammer bei 20 bis 22°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Feuchtkammer gestellt.

6 Tage nach der Inokulation erfolgt die Auswertung.

Bei diesem Test zeigt z.B. die folgende Verbindung der Herstellungsbeispiele (2) bei einer Wirkstoffkonzentration von 100 ppm einen Wirkungsgrad von 100 %.

### Beispiel: B

### Uncinula-Test (Rebe) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung bis besprüht. Nach Antrocknen des Spritzbelages werden die Pflanzen mit Konidien des Pilzes Uncinula necator bestäubt. Die Pflanzen werden anschließend bei 23 bis 24°C und bei einer relativen Luftfeuchtigkeit von ca 75% im Gewächshaus aufgestellt.

14 Tage nach der Inokulation erfolgt die Auswertung.

Bei diesem Test zeigt z.B. die folgende Verbindung der Herstellungsbeispiele (2) bei einer Wirkstoffkonzentration von 10 ppm einen Wirkungsgrad von 100 %.

### Beispiel: C

### Sphaerotheca-Test (Gurke) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Bei diesem Test zeigt z.B. die folgende Verbindung (2) der Herstellungsbeispiele bei einer Wirkstoffkonzentration von 100 ppm einen Wirkungsgrad von 100 %.

### Beispiel D

### Venturia-Test (Apfel) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wässrigen Konidiensuspension des Apfelschorferregers Venturia inaequalis inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Bei diesem Test zeigt z.B. die folgende Verbindung der Herstellungsbeispiele (2) bei einer Wirkstoffkonzentration von 10ppm einen Wirkungsgrad von 100 %.

### Beispiel E

### Erysiphe-Test (Gerste) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Bei diesem Test zeigt z.B. die folgende Verbindung (2) bei einer Wirkstoffaufwandmenge von 250 g/ha einen Wirkungsgrad von 81 %.

### Beispiel F

### Leptosphaeria nodorum -Test (Weizen) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung.

Nach Antrocknen des Spritzbelages werden die Pflanzen mit einer Sporensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

Bei diesem Test zeigt z.B. die folgende Verbindung der Herstellungsbeispiele (2) bei einem Wirkstoffaufwandmenge von 250 g/ha einen Wirkungsgrad von 88 %.

## Patentansprüche

1. Verbindungen der allgemeinen Formel (Ia), 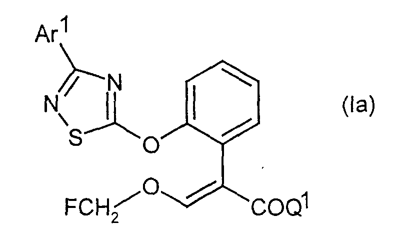 in welcher
| | | | |
|---|---|---|---|
| Ar¹ | für 4-Bromphenyl | und Q¹ | für Methoxy, oder |
| Ar¹ | für 4-Methylphenyl | und Q¹ | für Methoxy, oder |
| Ar¹ | für 4-Methoxyphenyl | und Q¹ | für Methoxy, oder |
| Ar¹ | für 4-Chorphenyl | und Q¹ | für Methoxy, oder |
| Ar¹ | für 4-Fluorphenyl | und Q¹ | für Methoxy, oder |
| Ar₁ | für 3-Methylphenyl | und Q¹ | für Methoxy, oder |
| Ar¹ | für 2-Methylphenyl | und Q¹ | für Methoxy, oder |
| Ar₁ | für 3-Fluorphenyl | und Q¹ | für Methoxy, oder |
| Ar¹ | für 2-Fluorphenyl | und Q¹ | für Methoxy, oder |
| Ar¹ | für 4-Bromphenyl | und Q¹ | für Ethoxy, oder |
| Ar¹ | für 2,4-Dimethylphenyl | und Q¹ | für Methoxy, oder |
| Ar¹ | für 3,4-Dichlorphenyl | und Q¹ | für Methoxy, oder |
| Ar₁ | für 4-Trifluormethylphenyl | und Q¹ | für Methoxy, oder |
| Ar₁ | für 3-Chlorphenyl | und Q¹ | für Ethoxy, oder |
| Ar¹ | für 2-Chlorphenyl | und Q¹ | für Methoxy, oder |
| Ar¹ | für 2-Bromphenyl | und Q¹ | für Methoxy, oder |
| Ar¹ | für 2-Methoxyphenyl | und Q¹ | für Methoxy, oder |
| Ar¹ | für 3-Chlorphenyl | und Q¹ | für Methoxy steht. |

2. Verfahren zur Herstellung von Verbindungen der Formel (I) gemäß Anspruch 1
**dadurch gekennzeichnet, daß** man Hydroxyarylverbindungen der allgemeinen Formel (II), 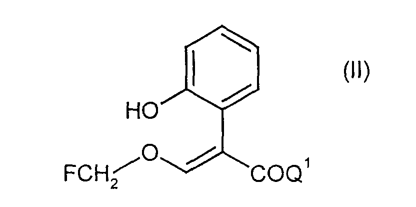 in welcher
Q¹ die in Anspruch 1 angegebenen Bedeutungen hat,
mit einemThiadiazolderivat der allgemeinen Formel (III), 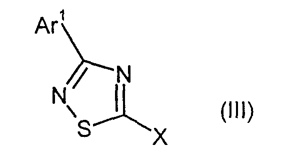 in welcher
Ar¹ die in Anspruch 1 angegebenen Bedeutungen hat und
X für Fluor, Chlor, Methylsulfonyl, Benzylsulfonyl oder Tolylsulfonyl steht,
gegebenenfalls in Gegenwart eines Säureakzeptors und gegebenenfalls in Gegenwart eines Verdünnungsmittels umsetzt.

3. Fungizides Mittel, **gekennzeichnet durch** einen Gehalt an mindestens einer Verbindung der Formel (I) nach Anspruch 1, mindestens einem Streckmittel und/oder mindestens einem oberflächenaktiven Mittel.

4. Verfahren zur Bekämpfung von Schädlingen, **dadurch gekennzeichnet, daß** man Verbindungen der Formel (I) nach Anspruch 1 auf Schädlinge und/oder ihren Lebensraum einwirken läßt.

5. Verwendung von Verbindungen der Formel (I) nach Anspruch 1 zur Bekämpfung von Schädlingen.

6. Verfahren zur Herstellung von fungiziden Mitteln, **dadurch gekennzeichnet, daß** man Verbindungen der Formel (I) nach Anspruch 1 mit Streckmitteln und/oder oberflächenaktiven Mitteln vermischt.

## Claims

1. Compounds of the general formula (Ia) 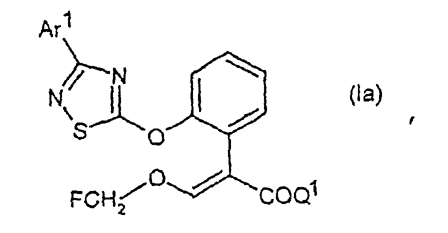 in which
Ar¹ represents bromophenyl and Q¹ represents methoxy, or
Ar¹ represents 4-methylphenyl and Q¹ represents methoxy, or
Ar¹ represents 4-methoxyphenyl and Q¹ represents methoxy, or
Ar¹ represents 4-chlorophenyl and Q¹ represents methoxy, or
Ar¹ represents 4-fluorophenyl and Q¹ represents methoxy, or
Ar¹ represents 3-methylphenyl and Q¹ represents methoxy, or
Ar¹ represents 2-methylphenyl and Q¹ represents methoxy, or
Ar¹ represents 3-fluorophenyl and Q¹ represents methoxy, or
Ar¹ represents 2-fluorophenyl and Q¹ represents methoxy, or
Ar¹ represents 4-bromophenyl and Q¹ represents ethoxy, or
Ar¹ represents 2,4-dimethylphenyl and Q¹ represents methoxy, or
Ar¹ represents 3,4-dichlorophenyl and Q¹ represents methoxy, or
Ar¹ represents 4-trifluorophenyl and Q¹ represents methoxy, or
Ar¹ represents 3-chlorophenyl and Q¹ represents ethoxy, or
Ar¹ represents 2-chlorophenyl and Q¹ represents methoxy, or
Ar¹ represents 2-bromophenyl and Q¹ represents methoxy, or
Ar¹ represents 2-methoxyphenyl and Q¹ represents methoxy, or
Ar¹ represents 3-chlorophenyl and Q¹ represents methoxy.

2. Process for the preparation of compounds of the formula (Ia), according to Claim 1,
**characterized in that** hydroxyaryl compounds of the general formula (II) 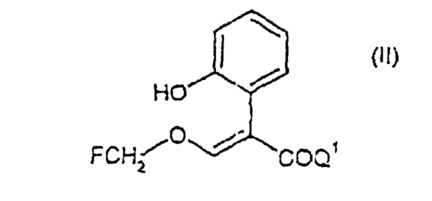 in which Q¹ has the meanings indicated im Claim 1,
are reacted with a thiadiazole derivative of the general formula (III) 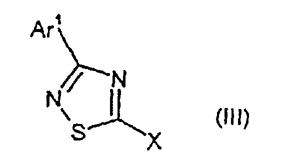 in which
Ar¹ has the meanings indicated in Claim 1 and
X represents fluorine, chlorine, methylsulphonyl. benzylsulphonyl or toluylsulphonyl,
if appropriate in the presence of an acid acceptor and if appropriate in the presence of a diluent.

3. Fungicidal composition, **characterized in that** it contains at least one compound of the formula (Ia) according to Claim 1, at least one extender and/or at least one surfactant.

4. Method of controlling pests, **characterized in that** compounds of the formula (Ia) according to Claim 1 are allowed to act on pests and/or their habitat.

5. Use of compounds of the formula (Ia) according to Claim 1 for controlling pests.

6. Process for the production of fungicidal agents, **characterized in that** compounds of the formula (Ia) according to Claim 1 are mixed with extenders and/or surface-active agents.

## Revendications

1. Composés de formule générale (Ia) : 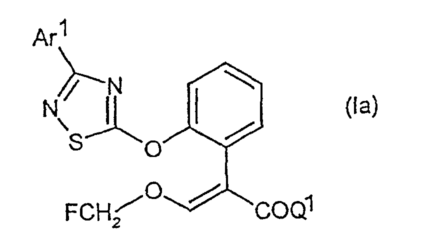 dans laquelle
Ar¹ est un groupe 4-bromophényle et Q¹ est un groupe méthoxy, ou
Ar¹ est un groupe 4-méthylphényle et Q¹ est un groupe méthoxy ou
Ar¹ est un groupe 4-méthoxyphényle et Q¹ est un groupe méthoxy, ou
Ar¹ est un groupe 4-chlorophényle et Q¹ est un groupe méthoxy, ou
Ar¹ est un groupe 4-fluorophényle et Q¹ est un groupe méthoxy, ou
Ar¹ est un groupe 3-méthylphényle et Q¹ est un groupe méthoxy, ou
Ar¹ est un groupe 2-méthylphényle et Q¹ est un groupe méthoxy, ou
Ar¹ est un groupe 3-fluorophényle et Q¹ est un groupe méthoxy, ou
Ar¹ est un groupe 2-fluorophényle et Q¹ est un groupe méthoxy, ou
Ar¹ est un groupe 4-bromophényle et Q¹ est un groupe éthoxy, ou
Ar¹ est un groupe 2,4-diméthylphényle et Q¹ est un groupe méthoxy, ou
Ar¹ est un groupe 3,4-dichlorophényle et Q¹ est un groupe méthoxy, ou
Ar¹ est un groupe 4-trifluorométhylphényle et Q¹ est un groupe méthoxy, ou
Ar¹ est un groupe 3-chlorophényle et Q¹ est un groupe éthoxy, ou
Ar¹ est un groupe 2-chlorophényle et Q¹ est un groupe méthoxy, ou
Ar¹ est un groupe 2-bromophényle et Q¹ est un groupe méthoxy, ou
Ar¹ est un groupe 2-méthoxyphényle et Q¹ est un groupe méthoxy, ou
Ar¹ est un groupe 3-chlorophényle et Q¹ est un groupe méthoxy.

2. Procédé de production de composés de formule (I) suivant la revendication 1,
**caractérisé en ce qu'**on fait réagir des composés hydroxy-aryliques de formule générale (II) 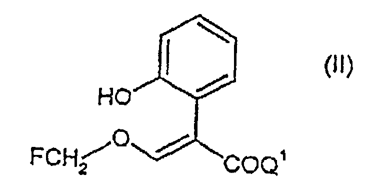 dans laquelle
Q¹ a les définitions indiquées dans la revendication 1,
avec un dérivé thiadiazolique de formule générale (III), 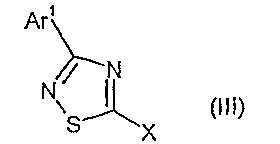 dans laquelle
Ar¹ a les définitions indiquées dans la revendication 1 et
X représente le fluor, le chlore, un groupe méthylsulfonyle, benzylsulfonyle ou tolylsulfonyle,
le cas échéant en présence d'un accepteur d'acide et en présence éventuelle d'un diluant.

3. Composition fongicide, **caractérisée par** une teneur en au moins un composé de formule (I) suivant la revendication 1, au moins un diluant et/ou au moins un agent tensioactif.

4. Procédé pour combattre des parasites, **caractérisé en ce qu'**on fait agir des composés de formule (I) suivant la revendication 1 sur les parasites et/ou sur leur milieu.

5. Utilisation de composés de formule (I) suivant la revendication 1 pour combattre des parasites.

6. Procédé de préparation de compositions fongicides, **caractérisé en ce qu'**on mélange des composés de formule (I) suivant la revendication 1 avec des diluants et/ou des agents tensioactifs.
